(19) Europäisches Patentamt
European Patent Office
Office européen des brevets

(11) Publication number: **0 135 956**
**A2**

(12) # EUROPEAN PATENT APPLICATION

(21) Application number: **84201150.4**

(22) Date of filing: **07.08.84**

(51) Int. Cl.⁴: **C 07 D 279/06**, C 07 D 277/10, C 07 D 281/02, A 01 N 43/86, A 01 N 43/78, A 01 N 43/72

(30) Priority: **26.08.83 GB 8323061**

(43) Date of publication of application: **03.04.85 Bulletin 85/14**

(84) Designated Contracting States: **AT BE CH DE FR GB IT LI LU NL SE**

(71) Applicant: **SHELL INTERNATIONALE RESEARCH MAATSCHAPPIJ B.V., Carel van Bylandtlaan 30, NL-2596 HR Den Haag (NL)**

(72) Inventor: **Harris, Martin, 54 Northwood Drive, Sittingbourne Kent (GB)**

(74) Representative: **Hunter, Keith Roger Ian et al, 4 York Road, London SE1 7NA (GB)**

(54) **Novel pesticidal heterocyclic compounds.**

(57) N-substituted heterocyclic compounds having insecticidal activity of the following general formula:

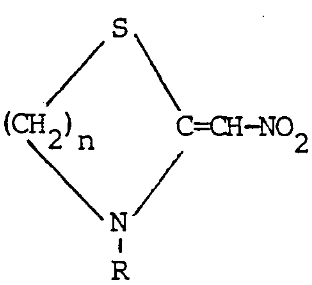

(I)

wherein n = 2, 3 or 4 and R is an optionally-substituted acyl group selected from the following:
aroyl
carbamoyl
aryloxycarbonyl
arylthiocarbonyl
alkylthio-thiocarbonyl
arylthio-thiocarbonyl
arylsulphonyl
alkylsulphinyl
arylsulphinyl
alkylaminosulphonyl
arylaminosulphonyl
alkylaminosulphinyl
arylaminosulphinyl
or a phosphorus-containing acyl group of the following formula:

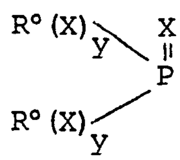

wherein each R° independently is an optionally substituted alkyl or aryl group, each X independently is an oxygen or sulphur atom and each y independently is 0 or 1.

EP 0 135 956 A2

0135956

K 1923 FF

"NOVEL PESTICIDAL HETEROCYCLIC COMPOUNDS"

This invention relates to novel N-substituted nitromethylene heterocyclic compounds, to a process for their preparation and to pesticidal compositions containing them.

Nitromethylene heterocyclic compounds are known to possess an interesting level of insecticidal activity - see for example UK Patent Specification 1.513.951 in which the following compound is particularly exemplified:-

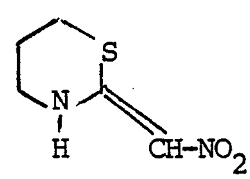

(A)

Hitherto N-substituted derivatives of Compound A and analogues thereof have been synthesised by derivatising the heterocyclic compound A or its analogues - see for example UK 1.513.951 page 5 lines 55-61. However attempts to synthesise certain N-acyl derivatives by the direct acylation of Compound A and its analogues failed to yield the desired derivatives. Moreover the synthesis of Compound A involved the use of hazardous intermediates and thus a route to N-acyl derivatives avoiding Compound A was desirable.

The Applicant has found a new process for the manufacture of N-substituted derivatives of Compound A and its analogues which process avoids the need to use the heterocyclic intermediate Compound A. As well as producing known compounds

this new process yields a class of insecticidally active novel compounds which form the subject of the present invention.

Accordingly the present invention provides N-substituted nitromethylene heterocyclic compounds of the following general formula:-

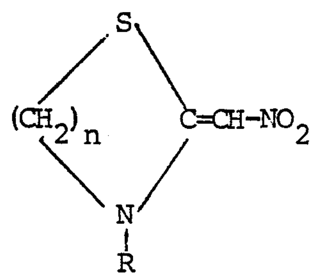

(I)

wherein n is 2,3 or 4 and R is an acyl group selected from the following groups each of which may be optionally substituted:-

aroyl,
carbamoyl,
aryloxycarbonyl,
alkylthiocarbonyl,
alkylthio-thiocarbonyl,
arylthio-thiocarbonyl,
arylsulphonyl,
alkylsulphinyl,
arylsulphinyl,
alkylaminosulphonyl,
arylaminosulphonyl,
alkylaminosulphinyl,
arylaminosulphinyl,
and a phosphorus-containing acyl group of the following formula:-

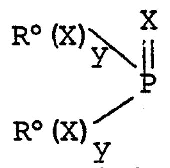

wherein each R° independently is an optionally substituted alkyl or aryl group, each X independently is an oxygen or sulphur atom and each y independently is 0 or 1.

Any aliphatic group present in an acyl group represented by R preferably contains up to 30 carbon atoms, more preferably up to 20 carbon atoms, often however the carbon content can be up to 8 or up to 4 carbon atoms. An aryl group present in the acyl group is preferably a phenyl group.

The optional substituents which may be present in an acyl group represented by R are preferably selected from fluorine, chlorine, bromine and iodine; nitro; cyano; alkyl, alkoxy, alkylthio, alkenyl and alkynyl preferably of up to 6 carbon atoms; and aryl, aryloxy and arylthio, preferably phenyl, phenoxy and phenylthio, optionally substituted with one or more groups selected from fluorine, chlorine, bromine, iodine C(1-4)alkyl, nitro and cyano.

Preferably n is 3.

More specifically the prefered compounds according to the invention have the general formula I in which n=3 and R is optionally substituted aroyl for example benzoyl or halobenzoyl, optionally substituted arylsulphonyl for example phenylsulphonyl or halophenylsulphonyl, dialkoxy-oxophosphorus or dialkoxy-thiophosphorus in which each alkyl group preferably contains 1 to 4 carbon atoms, diaryloxy-oxophosphorus wherein each aryl is preferably phenyl, or dialkylcarbamoyl or dialkylaminosulphonyl in which each alkyl group contains 1 to 4 carbon atoms.

It will be appreciated that the compounds of formula I are capable of existing in different geometrically isomeric forms. The invention includes the individual isomers and mixtures of such isomers.

The compounds according to the invention may be prepared by a new process which comprises reacting an N-substituted-S-substituted-aminothiol of the following general formula:-

$$R - NH - (CH_2)_n - S - \overset{Hal}{\overset{|}{C}} = CH - NO_2 \qquad (II)$$

wherein R and n have the meanings specified hereinbefore and Hal is a halogen atom, with a base, preferably one selected from an

alkali metal or alkaline earth metal hydride or alkoxide or an alkali metal amide, in the presence of an inert solvent. Suitable bases for use in this process are sodium or potassium hydride, sodium or potassium alkoxide in which the alkyl group contains 1 to 8 carbon atoms and is preferably branched e.g. a tertiary alkyl group, and sodium, potassium or lithium amides or dialkylamides (wherein each alkyl group contains 1 to 8 carbons atoms) e.g. sodamide and sodium di-isopropylamide.

Any suitable solvent may be used, for example tertiary alcohols e.g. tertiary-butanol, ethers e.g. diethyl ether, tetrahydrofuran, and dimethoxyethane, ketones e.g. acetone, nitrogenous solvents e.g. dimethylformamide, hydrocarbons e.g. benzene, xylene, and toluene, and sulphur-containing solvents, e.g. dimethyl sulphoxide.

The reaction may proceed at room temperature but in general it can take place at temperatures between -30° and + 50°C, suitably between -20° and +30°C.

The molar ratio of the reactants is unimportant, for example the molar ratio of base to aminothiol can be in the range 1:2 to 2:1 but generally speaking it is preferred to have the basic reactant in excess, e.g. the molar ratio is preferably in the range 1:1 to 2:1.

The N-substituted-S-substituted aminothiol starting materials of general formula II in the process according to the invention are novel compounds. They may be prepared by a process which comprises reacting an aminothiol of the general formula

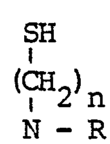

(III)

wherein R and n have the meanings hereinbefore specified with a halonitroethylene of the general formula

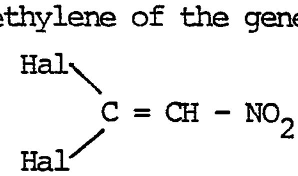

(IV)

wherein Hal is a halogen atom in the presence of a base, preferably a weaker base than that used in the preparation of the heterocyclic compound of general formula I, for example a hydroxide, carbonate, bicarbonate or acetate of an alkali or alkaline earth metal or of ammonium and in the presence of an inert solvent. Conveniently the base is an alkali metal hydroxide such as sodium or potassium hydroxide. The inert solvent can be any of the solvents referred to hereinbefore.

It is generally preferred to isolate the aminothiol of general formula II before subjecting it to the action of a base to form the heterocyclic compound I. However isolation of the intermediate II is inessential and the heterocyclic compound can be made directly without isolation of the intermediate II by reacting the aminothiol III with the halonitroethylene IV in the presence of base.

The aminothiol compounds of general formula III may be prepared by N-acylation of the corresponding unsubstituted aminothiol.

As mentioned above the N-substituted nitromethylene heterocyclic compounds of the invention are of interest as pesticides particularly against insect pests. They exhibit activity against such pests as the larval caterpillar or worm forms of insects, for examples, of the genus Spodoptera and of the genus Heliothis, and are particularly useful against pests found in rice crops. The compounds also exhibit acceptable stability towards light and oxidation.

Accordingly the invention includes a pesticidal composition comprising an N-substituted nitromethylene heterocyclic compound of general formula I together with a carrier.

Such a composition may contain a single compound or a mixture of several compounds of the invention. It is also envisaged that different isomers or mixtures of isomers may have different levels or spectra of activity and thus compositions may comprise individual isomers or mixtures of isomers. The invention further provides a method of combating pests at a

locus, particular insect pests at a locus infested, or liable to infestation, by such pests, which comprises applying to the locus a pesticidally effective amount of compound or composition according to the present invention.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose oe pentaerythritol; condesnates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The composition of the invention may, for example, be formulated as a wettable powder, a dust, granules, a solution, an emulsifiable concentrate, an emulsion, a suspension concentrate or an aerosol. The composition may have controlled release properties.

Wettable powders usually contain 25, 50 or 75% of active ingredient and may contain, in addition to inert solid material,

3-10%w of a dispersing agent and, where necessary, 0-10%w of a stabilizer, a penetrant and/or a sticker. A dust is usually formulated as a dust concentrate having a composition similar to that of a wettable powder but without a dispersant, and is diluted in the field with further solid carrier to give a composition usually containing ½-10%w of active ingredient.

Granules usually have a size in the range of from 10 to 100 BS mesh (1.676 - 0.152 mm) and may be manufactured by agglomeration or impregnation techniques. Generally, granules will contain ½-75% w active ingredient and 0-10%w of additives, such as stabilizers, surfactants, slow release modifiers and binding agents.

Emulsifiable concentrates usually contain, in addition to a solvent, and, when necessary, co-solvent, 10-50% w/v active ingredients, 2-20% w/v emulsifier and 0-20% w/v of other additives, for example a stabilizer, a penetrant and/or a corrosion inhibitor. A suspension concentrate is a stable, non-sedimenting, flowable product and usually contains 10-75%w active ingredient, 0.5-15%w of dispersing agent, 0.1-10% w of suspending agent, for example protective colloid and/or a thixotropic agent, and 0-10%w of other additives including, for example, a defoamer, a corrosion inhibitor, a stabilizer, a penetrant and/or a sticker, and as dispersant, water or an organic liquid in which the active ingredient is substantially insoluble; certain organic additives and/or inorganic salts may be dissolved in the dispersant to assist in preventing sedimentation or as anti-freeze for water.

The aqueous dispersions and emulsions formed by diluting a wettable powder or an emulsifiable concentrate of the invention with water, also lie within the socpe of the present invention. Such dispersions and emulsions may be of the water-in-oil or of the oil-in-water type, and may have a thick "mayonnaise"-like consistency.

A composition of the invention may also contain other ingredients, for example, one or more other compounds possessing

pesticidal properties. Further insecticidal compounds may be included, particularly such compounds having a different mode or spectrum of activity.

The thermal stability of the compounds and compositions of the invention may be improved by the addition of stabilizing amounts, usually 10-100%w based on the compound of certain organo nitrogen compounds such as urea, dialkylureas, thiourea or guanidine salts or alkali metal salts of weak acids such as bicarbonates, acetates or benzoates.

The invention is illustrated further in the following Examples.

Example 1 Preparation of N-benzoyl-2-nitromethylene-2H-1,3-thiazine

To N-benzoyl-3-aminopropanethiol (780mg) in methanol (12ml) at 0°C under nitrogen was added dropwise sodium hydroxide (160mg) in methanol (6ml). This mixture was then added dropwise at 0°C under nitrogen to 1,1-dichloro-2-nitroethene (1.2g) in methanol (20ml) over 20 minutes. The reaction mixture was poured into 2% HCl saturated with NaCl and extracted with methylene chloride. The product was purified using a silica gel column. Yield 252mg.

| | | | | |
|---|---|---|---|---|
| Analysis | Calculated: | C 47.9; | H 4.3; | N 9.3% |
| | Found | C 48.3; | H 4.3; | N 9.3% |

To N-benzoyl-S-(1-chloro-2-nitroethenyl)-3-aminopropanethiol(233mg) in t-butanol (10ml) under nitrogen was added dropwise potassium t-butoxide (100mg) in t-butanol(3ml) at room temperature over 5 minutes. After 40 minutes the reaction mixture was poured into 2% HCl saturated with NaCl and extracted with methylene chloride. The product was purified on a silica gel column. Yield 60 mg; m.p. 90-92°C.

| | | | | |
|---|---|---|---|---|
| Analysis: | Calculate : | C 54.5; | H 4.5; | N 10.6% |
| | Found : | C 53.8; | H 5.2; | N 10.1% |

Example 2A Preparation of N-phenylsulphonyl-2-nitromethylen-2H-1,3-thiazine

To the hydrochloride salt of 3-amino-propanethiol (5g) was added sodium methoxide (Na1g; methanol 25ml) and the mixture allowed to cool. The solvent was removed. Methylene chloride (60ml) and triethylamine (6g) were added to the product and phenylsulphonyl chloride (7g) in methylene chloride (40ml) added dropwise thereto at 0°C over 30 minutes. After 20 minutes at room temperature the product was washed with 2% HCl and triturated with diethyl ether. Yield 6.2g of the disulphide, bis-(N-phenylsulphonyl-3-aminopropyl)-disulphide.

The disulphide (3.1g) was treated with triphenylphosphine (2.5g) in water (2.5g), methanol (50ml) and 10 drops of 10% HCl and refluxed for 11 hours. Most of the solvent was removed and the resulting product was taken up in methylene chloride and washed with water. The product was purified over a silica gel column. Yield of N-phenylsulphonyl-3-aminopropanethiol 1.6g.

| | | | | |
|---|---|---|---|---|
| Analysis: | Calculated: | C 46.8; | H 5.6; | N 6.1% |
| | Found : | C 46.8; | H 5.8; | N 5.9% |

To N-phenylsulphonyl-3-aminopropanethiol (231mg) in methanol (5ml) sodium hydroxide (80mg) in methanol (3ml) was added over 5 minutes and this mixture was then added dropwise to 1,1-dichloro-2-nitroethene (284mg) in methanol (6ml) at 0°C over 20 minutes. The reaction mixture was maintained at 0°C for 30 minutes and then poured into 2% HCl saturated with NaCl and extracted with methylene chloride. The product was purified over a silica gel column. Yield 93mg; m.p. 105-106°C.

| | | | | |
|---|---|---|---|---|
| Analysis | Calculated: | C 39.2; | H 3.9; | N 8.3% |
| | Found: | C 39.6; | H 3.8; | N 8.2% |

To N-phenylsulphonyl-S-(1-chloro-2-nitroethenyl) 3-amino-propanethiol (100mg) in t-butanol(5ml) was added potassium t-butoxide (35mg) in t-butanol (2ml) over 6 minutes. After 20 minutes at room temperature the reaction mixture was poured into 2% HCl saturated with NaCl and then extracted with methylene chloride. The produce was purified on a silica gel column. Yield 54mg; m.p. 107°C.

Analysis: Calculated: C 44.0; H 4.0; N 9.2
Found : C 44.0; H 4.0; N 9.3

Example 2B Preparation of N-phenylsulphonyl-2-nitromethylene-2H-1,3-thiazine without isolating the N-substituted-S-substituted-aminothiol

To N-phenylsulphonyl-3-aminopropanethiol (231mg) in t-butanol (5 ml) was added potassium t-butoxide (224mg) dropwise over 10 minutes under nitrogen. This mixture was then added dropwise to 1,1-dichloro-2-nitroethene (300 mg) under nitrogen over 20 minutes. After 20 mintues at room temperature the reaction mixture was poured ito 2% HCl saturated with NaCl, and extracted with methylene chloride. The product was purified on a silica gel column using methylene chloride as eluant. Yield 31mg. m.p. 106-107°C.

Analysis: Calculated : C 44.0; H 4.0; N 9.3%
Found : C 43.9; H 4.0; N 9.2%

Example 3 Preparation of N-(diethoxythiophosphorus)-2-nitromethylene-2H-1,3-thiazine

To N-(diethoxythiophosphorus)-S-(1-chloro-2-nitroethenyl)-3-aminopropanethiol (180mg) in tertiary-butanol (10ml) was added potassium t-butoxide (65mg) in t-butanol (4ml) dropwise over 5 minutes. After 30 minutes at room temperature the reaction mixture was poured into 2% HCl saturated with NaCl and extracted with methylene chloride. The product was purified on a silica gel column using methylene chloride as eluant. Yield 76 mg; oil.

Example 4 to 6

By methods analogous to those described in Examples 1 to 3, the compounds detailed in Table I were prepared.

TABLE I

| Example No | In the General Formula I n | R | M.Pt °C | Elemental Analaysis | C | H | N |
|---|---|---|---|---|---|---|---|
| 4 | 3 | p-bromophenylsulphonyl | 91-92 | Calc: | 34.8 | 2.9 | 7.4 |
| | | | | Found: | 34.9 | 2.8 | 7.3 |
| 5 | 3 | $(CH_3)_2N.CO-$ | 104-106 | Calc: | 41.6 | 5.6 | 18.2 |
| | | | | Found: | 41.6 | 5.4 | 17.0 |
| 6 | 3 | $(CH_3)_2N.SO_2-$ | 92-93 | Calc: | 31.5 | 4.9 | 15.7 |
| | | | | Found: | 31.7 | 4.9 | 15.6 |

Example 7 Pesticidal Activity

The pesticidal activities of the compounds of the invention were assesed employing the following insect pests. The tests were all conducted under normal insectary conditions 23°C ± 2°C (fluctuating lght and humidity).

(i) *Spodoptera littoralis* (S.1)

Second instar larvae were used in the tests. A 0.2% solution or suspension of each test compound in 16.7% acetone in water containing 0.04% Triton X-100 (Trade Mark) was sprayed onto a separate petri dish containing a nutrious diet on which the *Spodoptera littoralis* larvae had been reared. Controls were sprayed with a control solution of water, acetone and Triton X-100 (Trade Mark) in the same proportions.

When the spray deposit had dried each dish was infested with 10 2nd instar larvae. Mortality assessments were made 1 and 7 days after spraying the percentage mortality calculated.

(ii) *Aedes aegypti* (A.a)

Early 4th instar larvae were used in the tests. Test solutions were made up to 3ppm of active ingredient in water containing 0.04% Triton X-100 (Trade Mark); acetone was initially present to aid solution, but was subsequently allowed to evaporate off.

Ten early 4th instar larvae were placed in 100ml of the test solution. After 48 hours, larval mortality (as a percentage) was recorded.

Any surviving larvae were then fed with a small quantity of animal feed pellets and the final percentage mortality of adults and pupae made when all the larvae had either pupated and turned into adults, or died.

The results of these tests are shown in Table II in which the test species are identified by the initials noted above and the activity of each compound is expressed in terms of the percentage mortality:

A denotes 90-100% mortality
B denotes 50-80% mortality
C denotes 0-40% mortality

TABLE II

| Compound of Example No. | S.l. | | A.a. | |
|---|---|---|---|---|
| | 24 hr | 7 day | 24 hr | Final |
| 1 | A | A | A | A |
| 2 | A | A | A | A |
| 3 | A | A | A | A |
| 4 | B | A | A | A |
| 5 | A | A | C | C |
| 6 | A | A | A | A |

K 1923 FF *

CLAIMS

1. An N-substituted heterocyclic compound of the general formula:-

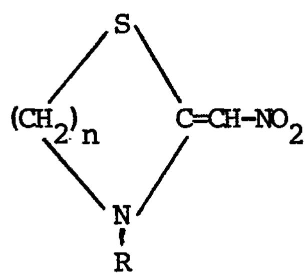

(I)

wherein n=2, 3 or 4 and R is an optionally substituted acyl group selected from the following:

aroyl
carbamoyl
aryloxycarbonyl
arylthiocarbonyl
alkylthio-thiocarbonyl
arylthio-thiocarbonyl
arylsulphonyl
alkylsulphinyl
arylsulphinyl
alkylaminosulphonyl
arylaminosulphonyl
alkylaminosulphinyl
arylaminosulphinyl

or a phosphorus-containing acyl group of the following formula:

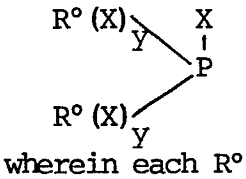

wherein each R° independently is an optionally-substituted alkyl or aryl group, each X independently is an oxygen or sulphur atom and each y independently is 0 or 1.

2. A compound according to claim 1 in which n is 3.

3. A compound according to either claim 1 or claim 2, in which any aliphatic group present in R has up to 8 carbon atoms, any aryl group is a phenyl group, and any optional substituents are selected from fluorine, chlorine, bromine and iodine; nitro; cyano; alkyl, alkoxy, alkylthio, alkenyl and alkynyl of up to 6 carbon atoms; and phenyl, phenoxy and phenylthio optionally substituted by one or more groups selected from fluorine, chlorine, bromine, iodine, C(1-4) alkyl, nitro and cyano.

4. A compound according to claim 1 in which n is 3 and R is benzoyl, halobenzoyl, phenylsulphonyl, halophenylsulphonyl, dialkyloxy-oxophosphorus, dialkyloxy-thiophosphorus, diphenoxy-oxophosphorus, or dialkylcarbamoyl or dialkylaminosulphonyl in which each alkyl group has 1 to 4 carbon atoms.

5. A pesticidal composition comprising as active ingredient a compound according to any one of the claims 1 to 4 together with a carrier.
6. A composition according to claim 5, which comprises at least two carriers, at least one of which is a surface-active agent.
7. A composition according to either claim 5 or claim 6 characterised in that a thermal stabilizer is also present.
8. A method of combating pests at a locus, which comprises applying to the locus a pesticidally effective amount of a compound according to any one of claims 1 to 4 or a composition according to any one of claims 5 to 7.